(19) Europäisches Patentamt
European Patent Office
Office européen des brevets

(11) Veröffentlichungsnummer: **0 251 011**
**B1**

(12) # EUROPÄISCHE PATENTSCHRIFT

(45) Veröffentlichungstag der Patentschrift: **24.10.90**

(51) Int. Cl.[5]: **C 07 D 209/22,** A 61 K 31/405

(21) Anmeldenummer: **87108590.8**

(22) Anmeldetag: **15.06.87**

(54) **Indolylpropionsäuren.**

(30) Priorität: **28.06.86 DE 3621761**

(43) Veröffentlichungstag der Anmeldung:
**07.01.88 Patentblatt 88/01**

(45) Bekanntmachung des Hinweises auf die Patenterteilung:
**24.10.90 Patentblatt 90/43**

(84) Benannte Vertragsstaaten:
**AT BE CH DE ES FR GB IT LI NL SE**

(56) Entgegenhaltungen:
**EP-A-0 155 828**

**CHEMICAL ABSTRACTS, Band 94, 11. - 25. Mai 1981, Columbus, Ohio, USA D. LAGIDZE et al.: "Synthesis of some new analogs of melatonin and beta-carboline from 4-phenylpentanoic acid" Seite 637, Spalte 1, Zusammenfassung Nr. 156681z**

(73) Patentinhaber: **BAYER AG**
**D-5090 Leverkusen 1 Bayerwerk (DE)**

(72) Erfinder: **Rosentreter, Ulrich, Dr.**
**Kondorweg 23**
**D-5600 Wuppertal 1 (DE)**
Erfinder: **Böshagen, Horst, Dr.**
**Wiesenstrasse 4**
**D-5657 Haan (DE)**
Erfinder: **Lieb, Folker, Dr.**
**Alfred-Kubin Strasse 1**
**D-5090 Leverkusen 1 (DE)**
Erfinder: **Oediger, Hermann, Dr.**
**Roggendorfstrasse 51**
**D-5000 Koeln 80 (DE)**
Erfinder: **Fiedler, Volker-Bernd, Dr.**
**Lehner Mühle 46**
**D-5090 Leverkusen 3 (DE)**
Erfinder: **Perzborn, Elisabeth, Dr.**
**Am Tescher Busch 13**
**D-5600 Wuppertal 11 (DE)**
Erfinder: **Seuter, Friedel, Dr.**
**Moospfad 16**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 251 011 B1

Courier Press, Leamington Spa, England.

**Beschreibung**

Indolylpropionsäuren

Die Erfindung betrifft neue Indolylpropionsäuren, Verfahren zu ihrer Herstellung und ihre Verwendung in Arzneimitteln.

Gegenstand der Erfindung sind neue Indolylpropionsäuren der allgemeinen Formel (I)

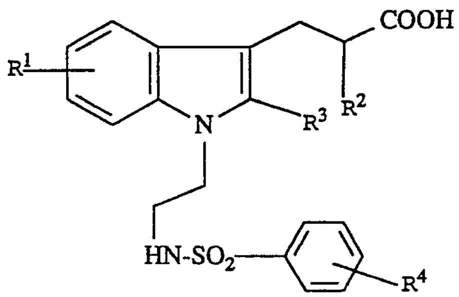

(I)

in welcher

$R^1$ für Wasserstoff, Niederalkyl, Niederalkoxy mit jeweils 1 bis 6 C-Atomen, Fluor, Chlor, Brom, Iod, Trifluormethyl, Trifluormethoxy, Phenyl, Phenoxy, Benzyl, Phenethyl, Phenoxy, Benzyloxy, Acetyl oder für eine Gruppe der Formel

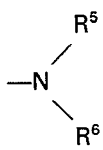 steht,

worin $R^5$ und $R^6$ gleich oder verschieden sind und Wasserstoff, Niederalkyl mit 1 bis 6 C-Atomen, Phenyl, Benzyl oder Acetyl bedeuten,

$R^2$ für Wasserstoff, Niederalkyl mit 1 bis 6 C-Atomen steht,

$R^3$ für Wasserstoff, Niederalkyl mit 1 bis 6 C-Atomen oder Phenyl steht,

und

$R^4$ für Wasserstoff, Niederalkyl, Niederalkoxy mit jeweils 1 bis 6 C-Atomen, Fluor, Chlor, Brom, , Trifluormethoxy, Phenyl, Phenoxy, Benzyl, Phenethyl, Benzyloxy, Acetyl oder für eine Gruppe der Formel

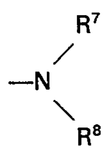 steht,

worin $R^7$ und $R^8$ gleich oder verschieden sind und Wasserstoff, Niederalkyl mit 1 bis 6 C-Atomen, Phenyl, Benzyl oder Acetyl bedeuten, und deren Salze.

Die erfindungsgemäßen Indolylpropionsäuren können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der Indolylpropionsäuren können Metall- oder Ammoniumsalze sein. Besonders bevorzugt sind z.B. Natrium-, Kalium-, Magnesium- oder Calciumsalze sowie Ammoniumsalze, die abgeleitet sind von Ammoniak oder organischen Aminen, wie beispielsweise Ethylamin, Di- bzw. Triethylamin, Di- bzw. Triethanolamin, Dicyclohexylamin, Dimethylaminoethanol, Arginin oder Ethylendiamin.

Die erfindungsgemäßen Stoffe zeigen überraschenderweise eine thrombozytenaggregationshemmende Wirkung und können zur therapeutischen Behandlung von Menschen und Tieren verwendet werden.

Besonders bevorzugt sind solche Verbindungen der allgemeinen Formel (I), in welcher

$R^1$ für Wasserstoff, $C_1$—$C_4$-Alkyl, $C_1$—$C_4$-Alkoxy, Fluor, Chlor, Brom, Trifluormethyl, $C_1$—$C_4$-Alkylamino oder Di-$C_1$—$C_4$-alkylamino steht,

$R^2$ für Wasserstoff oder $C_1$—$C_4$-Alkyl steht,

$R^3$ für Wasserstoff, $C_1$—$C_4$-Alkyl oder Phenyl steht,

und

$R^4$ für Wasserstoff, $C_1$—$C_4$-Alkyl, $C_1$—$C_4$-Alkoxy, Fluor, Chlor, Brom, Trifluormethyl, $C_1$—$C_4$-Alkylamino oder Di-$C_1$—$C_4$-alkylamino steht, und deren Salze.

Beispielhaft seien folgende Indolylpropionsäuren genannt:
3-{1-[2-[(4-Methylphenyl)sulfonamido]ethyl]indol-3-yl}-propionsäure
3-{1-[2-[(4-Fluorphenyl)sulfonamido]ethyl]indol-3-yl}-propionsäure
3-{1-[2-[(4-Chlorphenyl)sulfonamido]ethyl]indol-3-yl}-propionsäure
3-{2-Methyl-1-[2-[(4-fluorphenyl)sulfonamido]ethyl]indol-3-yl}-propionsäure
3-{2-Methyl-1-[2-[(4-chlorphenyl)sulfonamido]ethyl]indol-3-yl}-propionsäure
3-{5-Chlor-1-[2-[(4-fluorphenyl)sulfonamido]ethyl]indol-3-yl}-propionsäure
3-{6-Methoxy-1-[2-[(4-chlorphenyl)sulfonamido]ethyl]indol-3-yl}-propionsäure
3-{1-[2-[(4-Chlorphenyl)sulfonamido]ethyl]-2-phenyl-indol-3-yl}-propionsäure

Es wurde weiterhin ein Verfahren zur Herstellung von Indolylpropionsäuren er allgemeinen Formel (I)

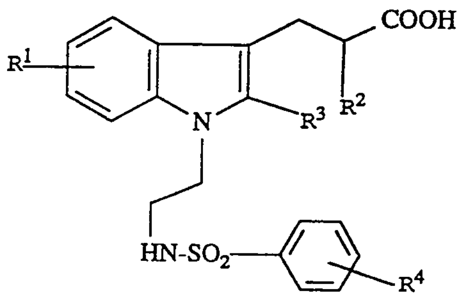

(I)

in welcher

$R^1$, $R^2$, $R^3$ und $R^4$ die oben angegebene Bedeutung haben, gefunden, das dadurch gekennzeichnet ist, daß man N-Sulfonamidoethyl-indole der allgemeinen Formel (II)

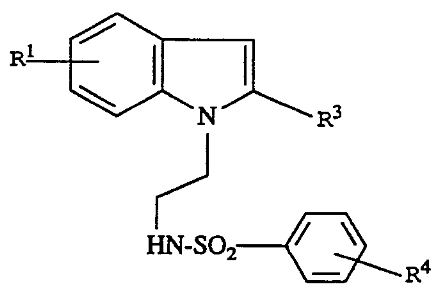

(II)

in welcher

$R^1$, $R^3$ und $R^4$ die angegebene Bedeutung haben, mit Acrylsäuren der allgemeinen Formel (III)

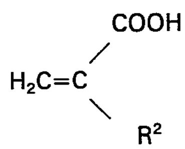

in welcher

$R^2$ die oben angegebene Bedeutung hat, in inerten Lösungsmitteln umsetzt, und im Fall der

Herstellung der Salze mit einer entsprechenden Base umsetzt. Das erfindungsgemäße Verfahren kann durch folgendes Formelschema erläutert werden:

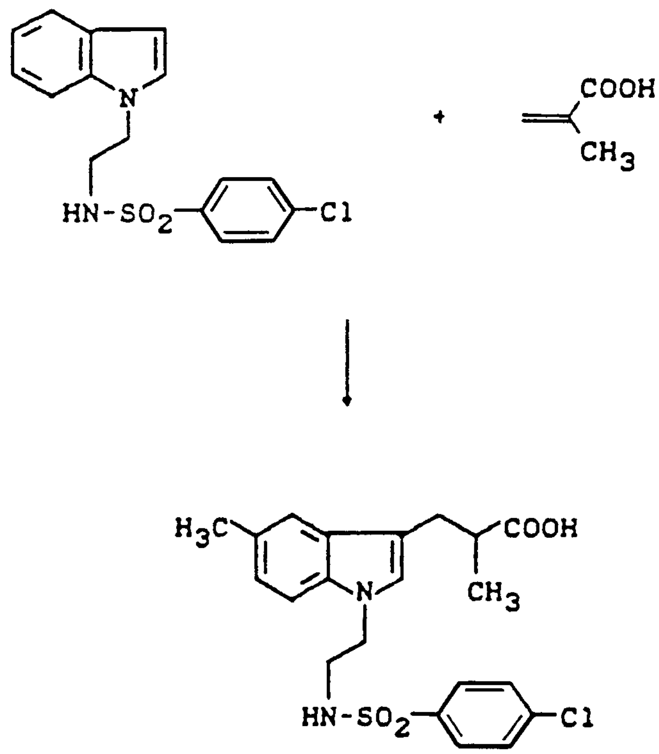

Als Lösungsmittel für das erfindungsgemäße Verfahren kommen inerte organische Lösungsmittel in Frage, die sich unter den Reaktionsbedingungen nicht verändern. Hierzu, gehören bevorzugt Carbonsäuren wie Ameisensäure, Essigsäure, Propionsäure, Trichloressigsäure oder Trifluoressigsäure, oder Carbonsäureanhydride wie Essigsäureanhydrid, Propionsäureanhydrid oder Trifluoracetanhydrid. Ebenso ist es möglich Gemische der genannten Lösungsmittel einzusetzen. Besonders bevorzugt wird ein Gemisch aus Carbonsäure und Carbonsäureanhydrid eingesetzt wie beispielsweise ein Gemisch aus Essigsäure und Acetanhydrid oder Trifluoressigsäure und Trifluoracetanhydrid.

Das Verfahren wird im allgemeinen in einem Temperaturbereich von 0°C bis +150°C, bevorzugt von +20°C bis +110°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Druckbereich von 0,5 bis 5 bar).

Im allgemeinen setzt man 1 bis 6 Mol, bevorzugt 2,5 bis 4 Mol der Acrylsäure (III) bezogen auf 1 Mol des N-Sulfonamidoethyl-inols (II) ein.

Das Überführen der Indolylpropionsäuren (I) in deren Salze erfolgt im allgemeinen durch Umsetzen der Säuren mit üblichen Basen gegebenenfalls in inerten Lösungsmitteln wie beispielsweise Wasser oder Alkohole z.B. Methanol, Ethanol, Propanol oder Isopropanol, oder Acetonitril, Dioxan, Tetrahydrofuran oder Dimethylformamid, oder deren Gemische.

Als Basen sind beispielsweise zu nennen: Alkali- oder Erdalkalioxide, -hydroxide, -carbonate oder -hydrogencarbonate zum Beispiel Natriumcarbonat, Kaliumcarbonat, Natriumhydrogencarbonat, Kaliumhydrogencarbonat, Natriumhydroxid, Kaliumhydroxid, Calciumhydroxid oder Magnesiumhydroxid oder Ammoniak oder organische Amine wie beispielsweise Ethylamin, Di-bzw. Triethylamin, Di-bzw. Triethanolamin, Dihexylamin oder Ethylendiamin.

Das erfindungsgemäße Verfahren kann beispielsweise wie folgt durchgeführt werden:

Das N-Sulfonamidoethyl-indol wird in einem inerten Lösungsmittel oder Lösungsmittelgemisch gelöst, mit Acrylsäure versetzt und gegebenenfalls erwärmt. Nach Beendigung der Reaktion wird durch Extraktion und/oder Chromatographie aufgearbeitet.

Als N-Sulfonamidoethyl-indole werden beispielsweise erfindungsgemäß eingesetzt:

N-[2-(Indol-1-yl)ethyl]-(4-methylphenyl)sulfonamid
N-[2-(Indol-1-yl)ethyl]-(4-fluorphenyl)sulfonamid
N-[2-(Indol-1-yl)ethyl]-(4-chlorphenyl)sulfonamid
N-[2-(2-Methylindol-1-yl)ethyl]-(4-fluorphenyl)sulfonamid
N-[2-(2-Methylindol-1-yl)ethyl]-(4-chlorphenyl)sulfonamid
N-[2-(5-Chlorindol-1-yl)ethyl]-(4-fluorphenyl)sulfonamid

N-[2-(2-Phenylindol-1-yl)ethyl]-(4-chlorphenyl)sulfonamid
N-[2-(6-Methoxyindol-1-yl)ethyl]-(4-chlorphenyl)sulfonamid.

Die Acrylsäuren der Formel (III) sind bekannt oder können nach bekannten Methoden hergestellt werden [Beilstein's Handbuch der organischen Chemie *2*, 397, 421].

Als Acrylsäuren können beispielsweise erfindungsgemäß verwendet werden:
Acrylsäure oder Methacrylsäure.

Die N-Sulfonamidoethyl-indole der allgemeinen Formel (II) sind neu.

Sie können hergestellt werden, indem man Indole der allgemeinen Formel (IV)

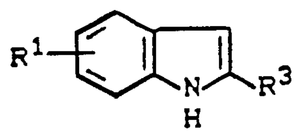
(IV)

in welcher
$R^1$ und $R^3$ die oben angegebene Bedeutung haben, mit Aziridinen der allgemeinen Formel (V)

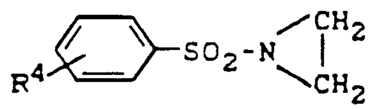
(V)

in welcher
$R^4$ die oben angegebene Bedeutung hat, in Gegenwart von Basen in inerten Lösungsmitteln umsetzt.

Die Herstellung der als Ausgangsstoffe verwendeten N-Sulfonamidoethyl-indole der allgemeinen Formel (II) läßt sich durch folgendes Formelschema verdeutlichen:

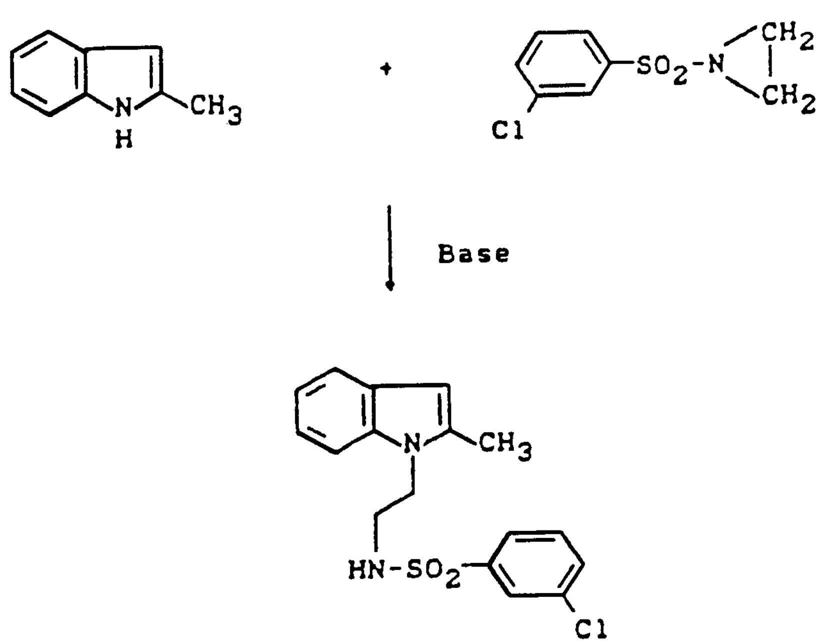


Als Lösungsmittel für das Verfahren können inerte organische Lösungsmittel verwendet werden, die sich unter den Reaktionsbedingungen nicht verändern. Hierzu gehören bevorzugt Ether wie beispielsweise Diethylether, Dioxan, Tetrahydrofuran oder Glykoldimethylether, oder Kohlenwasserstoffe wie beispielsweise Benzol, Toluol oder Xylol, oder Chlorkohlenwasserstoffe wie beispielsweise Dichlormethan, Trichlormethan, Tetrachlormethan oder Chlorbenzol, oder Amide wie beispielsweise Dimethylformamid oder Hexamethylphosphorsäuretriamid, oder Dimethylsulfoxid. Ebenso ist es möglich Gemische der genannten Lösungsmittel einzusetzen.

Als Basen eignen sich die üblichen basischen Verbindungen für basische Reaktionen. Hierzu gehören vorzugsweise Alkali- oder Erdalkalihydroxide wie beispielsweise Natriumhydroxid, Kaliumhydroxid oder Bariumhydroxid, oder Natrium- oder Kaliumcarbonate wie beispielsweise Natriumcarbonat, Natriumhydrogencarbonat oder Kaliumcarbonat, oder Alkalialkoholate wie beispielsweise Natriummethanolat, Kaliummethanolat, Natriumethanolat, Kaliumethanolat oder Kalium-tert-butanolat, oder Alkalihydride wie beispielsweise Lithiumhydrid, Natriumhydrid oder Kaliumhydrid, oder Alkaliamide wie beispielsweise Natriumamid, Kaliumamid oder Lithiumdiisopropylamid, oder lithiumorganische Verbindungen wie beispielsweise n-Butyllithium, tert-Butyllithium oder Phenyllithium, oder Natriumhexamethylsilazan.

Das Verfahen wird im allgemeinen in einem Temperaturbereich von −80°C bis +50°C, bevorzugt von −20°C bis +20°C durchgeführt.

Das Verfahren wird im allgemeinen bei Normaldruck durchgeführt. Es ist aber auch möglich, das Verfahren bei Unter- oder Überdruck durchzuführen (beispielsweise in einem Druckbereich von 0,5 bis 10 bar).

Im allgemeinen setzt man 1 bis 3 Mol, bevorzugt 1 bis 1,5 Mol der Base bezogen auf 1 Mol Indol ein. Das Indol wird im allgemeinen in einer Menge von 1 Mol bis 6 Mol, bevorzugt von 1 Mol bis 3 Mol bezogen auf 1 Mol des Aziridins eingesetzt.

Die Herstellung der N-Sulfonamidoethyl-indole kann beispielsweise wie folgt durchgeführt werden: Das Indol wird in einem Lösungsmittel gelöst und portionsweise mit einer Base versetzt. Danach wird das Aziridin zu der Reaktionslösung zugegeben. Die Aufarbeitung nach Beendigung der Reaktion erfolgt durch Extraktion und/oder Chromatographie.

Die als Ausgangsstoffe eingesetzten Indole der allgemeinen Formel (IV) sind bekannt oder können nach bekannten Methoden hergestellt werden [Houben-Weyls "Methoden der organischen Chemie" 10/2, 570ff].

Die als Ausgangsstoffe eingesetzten Aziridine der allgemeinen Formel (V) sind bekannt oder können nach bekannten Methoden aus Sulfonsäurechloriden und Ethylenimin hergestellt werden [Howard; Marckwald Chem. Ber. *32*, 2037].

Als Indole werden beispielsweise erfindungsgemäß verwendet:

Indol, 2-Methylindol, 2-Ethylindol, 2-Isopropylindol, 5-Chlorindol, 5-Fluorindol, 5-Methylindol, 5-Methoxyindol, 2-Phenylindol, 6-Methoxyindol, 6-Fluorindol, 6-Chlorindol, 5-Fluor-2-methyl-indol, 5-Chlor-2-methyl-indol.

Als Aziridine werden beispielsweise erfindungsgemäß verwendet:

N-(4-Methylphenyl)aziridin
N-(4-Fluorphenyl)aziridin
N-(4-Chlorphenyl)aziridin
N-(3-Methylphenyl)aziridin
N-(3-Fluorphenyl)aziridin
N-(3-Chlorphenyl)aziridin.

Die neuen Indolylpropionsäuren bzw. deren Salze können als Wirkstoffe in Arzneimitteln eingesetzt werden. Die Wirkstoffe weisen eine thrombozytenaggregationshemmende und Thromboxan$A_2$-antagonistische Wirkung auf. Sie können bevorzugt zur Behandlung von Thrombosen, Thromboembolien, Ischämien, als Antiasthmatika und als Antiallergika eingesetzt werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend, sind, um den angegebenen Dosierungsspielraum zu erreichen.

Die Formulierungen wrden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin-, Sulfitablaugen, Methylcellulose, Sträke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Tragermaterialien eingesetzt werden.

Im allgemeinen hat es sich als vorteilhaft erwiesen, bei intravenöer Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht. Trotzdem kann es gegebenenfalls erforderlich sein, von den genannten Mengen abzuweichen, und zwar in Abhängigkeit vom Körpergewicht bzw. der Art des

Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese im mehreren Einzelgaben über den Tag zu verteilen.

Herstellungsbeispiele

Beispiel 1

N-[2-(Indol-1-yl)ethyl]-(4-methylphenyl)sulfonamid

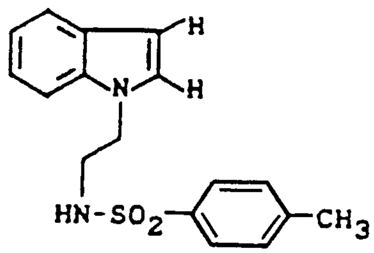

19,3 g (0,165 Mol) Indol werden in 50 ml Dimethylformamid gelöst und unter Stickstoff portionsweise mit 5,28 g (0,176 Mol) 80%iger Natriumhydridsuspension in Spindelöl versetzt. Nach Beendigung der Wasserstoffentwicklung werden bei 0°C 16,2 g (0,083 Mol) N-Tosylaziridin zur Reaktionslösung gegeben. Die Reaktionslösung wird 1 h bei 0°C weitergerührt und dann 1 h bei Raumtemperatur geruhrt. Anschließend wird mit Wasser vorsichtig verdünnt und mit Essigester extrahiert. Die Essigesterphase wird noch mehrmals mit Wasser gewaschen, mit Natriumsulfat getrocknet und eingedampft. Der Rückstand wird an Kieselgel (0,040 bis 0,063 mm, Merck) mit einem Gemisch von Toluol und Essigester im Verhältnis 9 zu 1 chromatographiert. Man erhält so eine Fraktion, die nach dem Eindampfen 19,3 g (74,5% der Theorie) festes Produkt ergibt.

$R_f$-Wert: 0,27 (Toluol:Essigester = 9:1)

Fp.: 128—130°C

Beispiel 2

3{1-[2-[(4-Methylphenyl)sulfonamido]ethyl]indol-3-yl}propionsäure

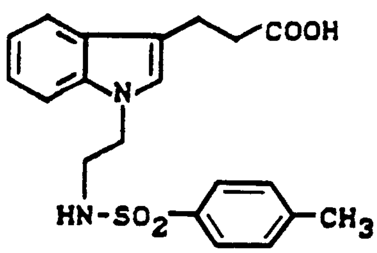

7,3 g (23,2 mmol) N-[2-(Indol-1-yl)ethyl]-(4-methylphenyl)sulfonamid werden in einem Gemisch von 14 ml Eisessig und 6 ml Acetanhydrid gelöst und mit 4,6 g ([63,9 mmol) Acrylsäure versetzt. Die Reaktionslösung wird 3 h bei 110°C gerührt. Nach dem Abkühlen wird mit Essigester verdünnt und mehrmals mit 2 n NaOH extrahiert. Die vereinigten wäßrigen Phasen werden mit Essigester extrahiert. Diese Essigesterphase wird mit Natriumsulfat getrocknet und eingedampft. Gründliches Trocknen im Hochvakuum ergibt 5,0 g (56% der Theorie) öliges Produkt. Dieses Öl wird in 30 ml Methanol gelöst und mit 0,7 g (13 mmol) Natriummethylat versetzt. Eindampfen im Vakuum und Kristallisation des Rückstandes aus Ether ergab 3,2 g kristallines Natriumsalz des Produkts.

$R_f$-Wert: 0,66 ($CH_2Cl_2/CH_3OH$ = 9/1)

Fp.: 125—135°C (Na-Salz)

Analog Beispiel 1 wurden die in Tabelle 1 aufgeführten Verbindungen hergestellt:

TABELLE 1

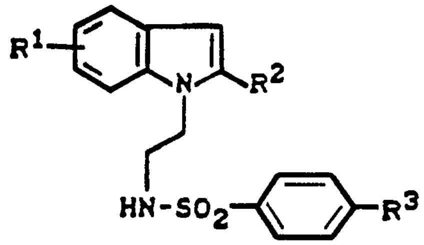

| Nr. | $R^1$ | $R^2$ | $R^3$ | $R_f$-Wert | Ausbeute [%] | Fp [°C] |
|---|---|---|---|---|---|---|
| 3 | H | H | —F | 0,45[a)] | 72,6 | Öl |
| 4 | H | $—CH_3$ | —F | 0,45[a)] | 76 | 110—112 |
| 5 | H | H | —Cl | 0,54[a)] | 76,8 | 84—86 |
| 6 | H | $—CH_3$ | —Cl | 0,56[a)] | 70 | 127—130 |
| 7 | 5—Cl | H | —F | 0,45[a)] | 55,4 | 95—98 |
| 8 | H | $—C_6H_5$ | —Cl | 0,63[b)] | 67 | 108—110 |
| 9 | $6—OCH_3$ | H | —Cl | 0,42[a)] | 75,3 | Öl |

a) Toluol/Essigester 8/2
b) Methylenchlorid

Analog zu Beispiel 2 wurden die in Tabelle 2 aufgeführten Verbindungen hergestellt:

TABELLE 2

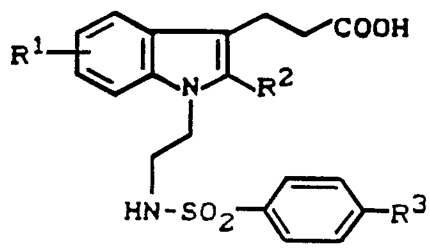

| Nr. | $R^1$ | $R^2$ | $R^3$ | $R_f$-Wert | Ausbeute [%] | Fp [°C] |
|---|---|---|---|---|---|---|
| 10 | H | H | —F | 0,32[c)] | 23,7 | 170 Na-Salz |
| 11 | H | $—CH_3$ | —F | 0,5[c)] | 49 | 170—90 Na-Salz |
| 12 | H | H | —Cl | 0,48[c)] | 100 | 230—40 Na-Salz |
| 13 | H | $—CH_3$ | —Cl | 0,45[c)] | 47 | 147—55 Na-Salz |
| 14 | 5—Cl | H | —F | 0,4[c)] | 14,7 | 45—50 Säure |
| 15 | H | $—C_6H_5$ | —Cl | 0,35[a)] | 17.6 | 70—80 Säure |
| 16 | $6—OCH_3$ | H | —Cl | 0,29[c)] | 9 | 184—5 Säure |

a) Toluol/Essigester 8/2
c) $CH_2Cl_2/CH_3OH$ 955

Anwendungsbeispiel

Zur Bestimmung der thrombozytenaggregationshemmenden Wirkung wurde Blut von gesunden Probanden beiderlei Geschlechts verwendet. Als antikoagulans wurden einem Teil 3,8%iger wäßriger Natriumzitratlösung 9 Teil Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Born, G.V.R., J. Physiol. (London), *162*, 67, 1962) im Aggregometer bei 37°C bestimmt (Therapeutische Berichte *47*, 80—86, 1975). Hierzu wurde die vorinkubierte Probe mit 0,1 ml Kollagen, einem agregationsauslösenden Agens, versetzt. Die Veränderung der optischen Dichte in der Probe der PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.

Als Grenzkonzentration wird der Bereich der minimal effektiven Konzentration angegeben (Tabelle 3).

TABELLE 3

| Beispiel-Nr | Hemmung mg/l (Grenzkonzentration) |
|---|---|
| 2 | 3—1 |
| 10 | 3—1 |
| 11 | 0,1—0,03 |
| 12 | 3—1 |
| 13 | 0,1—0,03 |

**Patentansprüche**

1. Indolylpropionsäuren der Formel

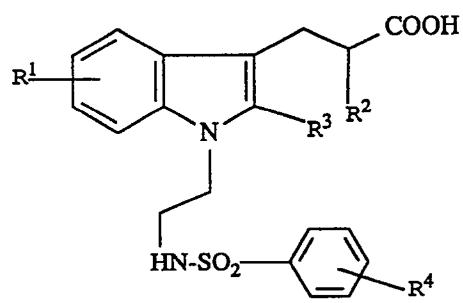

(I)

in welcher

$R^1$ für Wasserstoff, Niederalkyl, Niederalkoxy mit jeweils 1 bis 6 C-Atomen, Fluor, Chlor, Brom, Iod, Trifluormethyl, Trifluormethoxy, Phenyl, Phenoxy, Benzyl, Phenethyl, Phenoxy, Benzyloxy, Acetyl oder für eine Gruppe der Formel

$R^5$
/
—N steht,
\
$R^6$

worin

$R^5$ und $R^6$ gleich oder verschieden sind und Wasserstoff, Niederalkyl mit 1 bis 6 C-Atomen, Phenyl, Benzyl oder Acetyl bedeuten,

$R^2$ für Wasserstoff, Niederalkyl mit 1 bis 6 C-Atomen steht,

$R^3$ für Wasserstoff, Niederalkyl mit 1 bis 6 C-Atomen oder Phenyl steht,

und

$R^4$ für Wasserstoff, Niederalkyl, Niederalkoxy mit jeweils 1 bis 6 C-Atomen, Fluor, Chlor, Brom, , Trifluormethoxy, Phenyl, Phenoxy, Benzyl, Phenethyl, Benzyloxy, Acetyl oder für eine Gruppe der Formel

$R^7$
/
—N steht,
\
$R^8$

worin

$R^7$ und $R^8$ gleich oder verschieden sind und Wasserstoff, Niederalkyl mit 1 bis 6 C-Atomen, Phenyl, Benzyl oder Acetyl bedeuten, und deren Salze.

2. Indolylpropionsäuren nach dem Anspruch 1, worin

$R^1$ für Wasserstoff, $C_1$—$C_4$-Alkyl, $C_1$—$C_4$-Alkoxy, Fluor, Chlor, Brom, Trifluormethyl, $C_1$—$C_4$-Alkylamino oder Di-$C_1$—$C_4$-alkylamino steht,

$R^2$ für Wasserstoff oder $C_1$—$C_4$-Alkyl steht,

$R^3$ für Wasserstoff, $C_1$—$C_4$-Alkyl oder Phenyl steht,

und

$R^4$ für Wasserstoff, $C_1$—$C_4$-Alkyl, $C_1$—$C_4$-Alkoxy, Fluor, Chlor, Brom, Trifluormethyl, $C_1$—$C_4$-Alkylamino oder Di-$C_1$—$C_4$-alkylamino steht, und deren Salze.

3. Indolylpropionsäuren der Formel I gemäß Anspruch 1, zur Verwendung bei der therapeutischen Behandlung von Krankheiten.

4. Verfahren zur Herstellung von Indolylpropionsäuren der allgemeinen Formel (I)

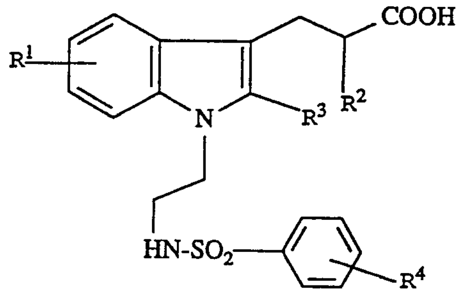

(I)

in welcher

$R^1$ für Wasserstoff, Niederalkyl, Niederalkoxy mit jeweils 1 bis 6 C-Atomen, Fluor, Chlor, Brom, Iod, Trifluormethyl, Trifluormethoxy, Phenyl, Phenoxy, Benzyl, Phenethyl, Phenoxy, Benzyloxy, Acetyl oder für eine Gruppe der Formel

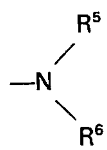

steht,

worin

$R^5$ und $R^6$ gleich oder verschieden sind und Wasserstoff, Niederalkyl mit 1 bis 6 C-Atomen, Phenyl, Benzyl oder Acetyl bedeuten,

$R^2$ für Wasserstoff, Niederalkyl mit 1 bis 6 C-Atomen steht,

$R^3$ für Wasserstoff, Niederalkyl mit 1 bis 6 C-Atomen oder Phenyl steht,

und

$R^4$ für Wasserstoff, Niederalkyl, Niederalkoxy mit jeweils 1 bis 6 C-Atomen, Fluor, Chlor, Brom, Trifluormethoxy, Phenyl, Phenoxy, Benzyl, Phenethyl, Benzyloxy, Acetyl oder für eine Gruppe der Formel

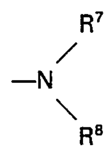

steht,

worin

$R^7$ und $R^8$ gleich oder verschieden sind und Wasserstoff, Niederalkyl mit 1 bis 6 C-Atomen, Phenyl, Benzyl oder Acetyl bedeuten, und deren Salze, das dadurch gekennzeichnet ist, daß man N-Sulfonamidoethyl-indole der allgemeinen Formel (II)

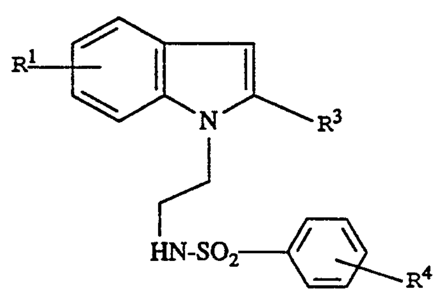

(II)

in welcher

$R^1$, $R^3$ und $R^4$ die angegebene Bedeutung haben, mit Acrylsäuren der allgemeinen Formel (III)

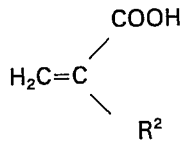

III

in welcher

$R^2$ die oben angegebene Bedeutung hat, in inerten Lösungsmitteln umsetzt, und im Fall der Herstellung der Salze mit einer entsprechenden Base umsetzt.

5. Arzneimittel, enthaltend Indolylpropionsäuren der Formel I gemäß Anspruch 1.

6. Verwendung von Indolylpropionsäuren der Formel I gemäß Anspruch 1 zur Herstellung von Arzneimitteln mit thrombozytenaggregationshemmender und/oder Thromboxan $A_2$-antagonistischer Wirkung.

7. N-Sulfonamidoethyl-indole der Formel

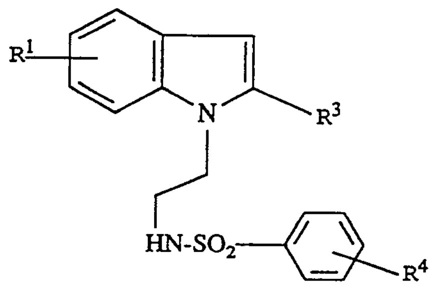

(II)

in der

$R^1$ für Wasserstoff, Niederalkyl, Niederalkoxy mit jeweils 1 bis 6 C-Atomen, Fluor, Chlor, Brom, Iod, Trifluormethyl, Trifluormethoxy, Phenyl, Phenoxy, Benzyl, Phenethyl, Phenoxy, Benzyloxy, Acetyl oder für eine Gruppe der Formel

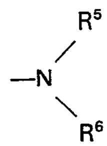 steht,

worin

$R^5$ und $R^6$ gleich oder verschieden sind und Wasserstoff, Niederalkyl mit 1 bis 6 C-Atomen, Phenyl, Benzyl oder Acetyl bedeuten,

$R^3$ für Wasserstoff, Niederalkyl mit 1 bis 6 C-Atomen oder Phenyl steht,

und

$R^4$ für Wasserstoff, Niederalkyl, Niederalkoxy mit jeweils 1 bis 6 C-Atomen, Fluor, Chlor, Brom, Trifluormethoxy, Phenyl, Phenoxy, Benzyl, Phenethyl, Benzyloxy, Acetyl oder für eine Gruppe der Formel

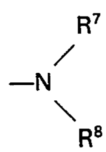 steht,

worin

$R^7$ und $R^8$ gleich oder verschieden sind und Wasserstoff, Niederalkyl mit 1 bis 6 C-Atomen, Phenyl, Benzyl oder Acetyl bedeuten.

8. Verfahren zur Herstellung von N-Sulfonamidoethylindolen der Formel

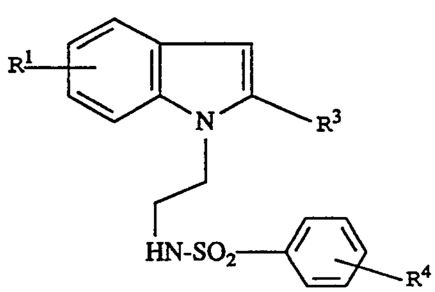

(II)

in der

$R^1$ für Wasserstoff, Niederalkyl, Niederalkoxy mit jeweils 1 bis 6 C-Atomen, Fluor, Chlor, Brom, Iod, Trifluormethyl, Trifluormethoxy, Phenyl, Phenoxy, Benzyl, Phenethyl, Phenoxy, Benzyloxy, Acetyl oder für eine Gruppe der Formel

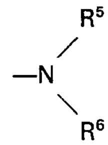 steht,

worin

$R^5$ und $R^6$ gleich oder verschieden sind und Wasserstoff, Niederalkyl mit 1 bis 6 C-Atomen, Phenyl, Benzyl oder Acetyl bedeuten,

$R^3$ für Wasserstoff, Niederalkyl mit 1 bis 6 C-Atomen oder Phenyl steht,

und

$R^4$ für Wasserstoff, Niederalkyl, Niederalkoxy mit jeweils 1 bis 6 C-Atomen, Fluor, Chlor, Brom, Trifluormethoxy, Phenyl, Phenoxy, Benzyl, Phenethyl, Benzyloxy, Acetyl oder für eine Gruppe der Formel

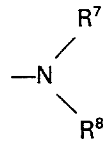 steht,

worin

$R^7$ und $R^8$ gleich oder verschieden sind und Wasserstoff, Niederalkyl mit 1 bis 6 C-Atomen, Phenyl, Benzyl oder Acetyl bedeuten, dadurch gekennzeichnet, daß man Indole der allgemeinen Formel (IV)

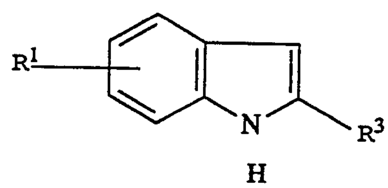

(IV)

in welcher

$R^1$ und $R^3$ die oben angegebene Bedeutung haben, mit Aziridinen der allgemeinen Formel (V)

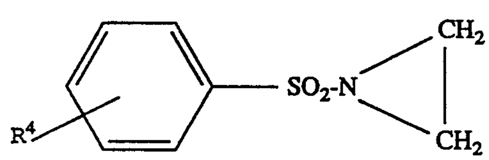

(V)

in welcher

$R^4$ die oben angegebene Bedeutung hat, in Gegenwart von Basen in inerten Lösungsmitteln umsetzt.

**Revendications**

1. Acides indolylpropioniques de formule

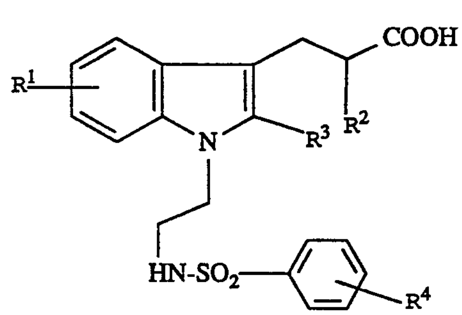

(I)

dans laquelle

$R^1$ représente l'hydrogène, un groupe alkyle inférieur, alkoxy inférieur ayant chacun 1 à 6 atomes de

carbone, le fluor, le chlore, le brome, l'iode, un groupe trifluorométhyle, trifluorométhoxy, phényle, phénoxy, benzyle, phénéthyle, phénoxy, benzyloxy, acétyle ou un groupe de formule

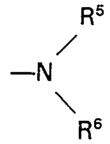

dans laquelle

$R^5$ et $R^6$ sont identiques ou différents et représentent l'hydrogène, un groupe alkyle inférieur ayant 1 à 6 atomes de carbone, phényle, benzyle ou acétyle,

$R^2$ est l'hydrogène, un groupe alkyle inférieur ayant 1 à 6 atomes de carbone,

$R^3$ est l'hydrogène, un groupe alkyle inférieur ayant 1 à 6 atomes de carbone ou le groupe phényle, et

$R^4$ représente l'hydrogène, un groupe alkyle inférieur, alkoxy inférieur ayant chacun 1 à 6 atomes de carbone, le fluor, le chlore, le brome, un groupe trifluorométhoxy, phényle, phénoxy, benzyle, phénéthyle, benzyloxy, acétyle ou un groupe de formule

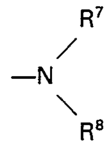

dans laquelle

$R^7$ et $R^8$ sont identiques ou différents et représentent l'hydrogène, un groupe alkyle inférieur ayant 1 à 6 atomes de carbone, phényle, benzyle ou acétyle, et leurs sels.

2. Acides indolylpropioniques suivant la revendication 1, dans lesquels

$R^1$ est l'hydrogène, un groupe alkyle en $C_1$ à $C_4$, alkoxy en $C_1$ à $C_4$, le fluor, le chlore, le brome, un groupe trifluorométhyle, alkylamino en $C_1$ à $C_4$ ou di(alkyle en $C_1$ à $C_4$)amino,

$R^2$ est l'hydrogène ou un groupe alkyle en $C_1$ à $C_4$,

$R^3$ est l'hydrogène, un groupe alkyle en $C_1$ à $C_4$ ou le groupe phényle,

et

$R^4$ est l'hydrogène, un groupe alkyle en $C_1$ à $C_4$, alkoxy en $C_1$ à $C_4$, le fluor, le chlore, le brome, un groupe trifluorométhyle, alkylamino en $C_1$ à $C_4$ ou di(alkyle en $C_1$ à $C_4$)amino, et leurs sels.

3. Acides indolylpropioniques de formule I suivant la revendication 1, destinés à être utilisés dans le traitement thérapeutique de maladies.

4. Procédé de production d'acides indolylpropioniques de formule générale (I)

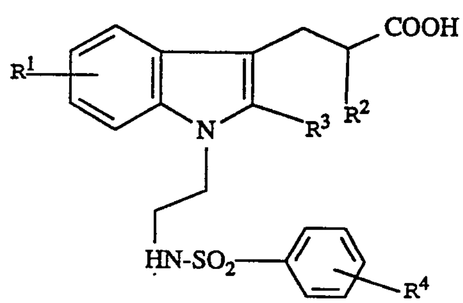

(I)

dans laquelle

$R^1$ représente l'hydrogène, un groupe alkyle inférieur, alkoxy inférieur ayant chacun 1 à 6 atomes de carbone, le fluor, le chlore, le brome, l'iode, un groupe trifluorométhyle, trifluorométhoxy, phényle, phénoxy, benzyle, phénéthyle, phénoxy, benzyloxy, acétyle ou un groupe de formule

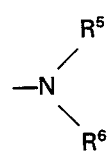

dans laquelle

$R^5$ et $R^6$ sont identiques ou différents et représentent l'hydrogène, un groupe alkyle inférieur ayant 1 à 6 atomes de carbone, phényle, benzyle ou acétyle,

$R^2$ est l'hydrogène, un groupe alkyle inférieur ayant 1 à 6 atomes de carbone,

$R^3$ est l'hydrogène, un groupe alkyle inférieur ayant 1 à 6 atomes de carbone ou le groupe phényle, et

$R^4$ représente l'hydrogène, un groupe alkyle inférieur, alkoxy inférieur ayant chacun 1 à 6 atomes de carbone, le fluor, le chlore, le brome, un groupe trifluorométhoxy, phényle, phénoxy, benzyle, phénéthyle, benzyloxy, acétyle ou un groupe de formule

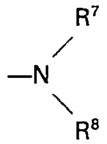

dans laquelle

$R^7$ et $R^8$ sont identiques ou différents et représentent l'hydrogène, un groupe alkyle inférieur ayant 1 à 6 atomes de carbone, phényle, benzyle ou acétyle, et de leurs sels, qui est caractérisé en ce qu'on fait réagir des N-sulfonamidoéthylindoles de formule générale (II)

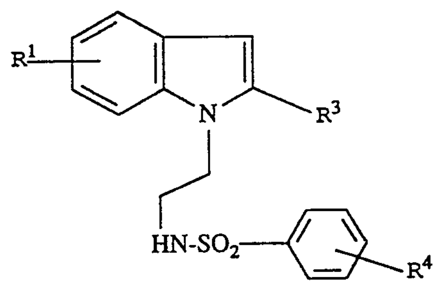

(II)

dans laquelle

$R^1$, $R^3$ et $R^4$ ont la définition indiquée ci-dessus, avec des acides acryliques de formule générale (III)

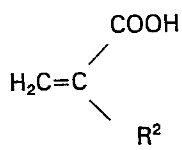

(III)

dans laquelle

$R^2$ a la définition indiquée ci-dessus dans des solvants inertes, et, dans le cas de préparations des sels, on les fait réagir avec une base correspondante.

5. Médicaments contenant des acides indolylpropioniques de formule I suivant la revendication 1.

6. Utilisation d'acides indolylpropioniques de formule I suivant la revendication 1 pour la préparation de médicaments ayant un effet inhibiteur d'agrégation des thrombocytes et/ou d'antagonisation du thromboxane $A_2$.

7. N-sulfonamidoéthylindoles de formule

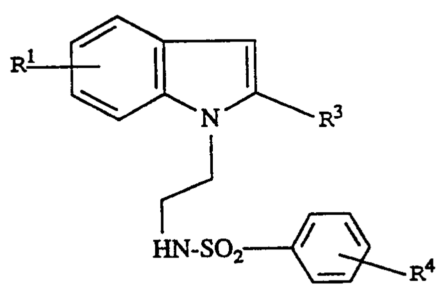

(II)

dans laquelle

$R^1$ représente l'hydrogène, un groupe alkyle inférieur, alkoxy inférieur ayant chacun 1 à 6 atomes de carbone, le fluor, le chlore, le brome, l'iode, un groupe trifluorométhyle, trifluorométhoxy, phényle, phénoxy, benzyle, phénéthyle, phénoxy, benzyloxy, acétyle ou un groupe de formule

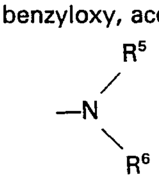

dans laquelle

$R^5$ et $R^6$ sont identiques ou différents et représentent l'hydrogène, un groupe alkyle inférieur ayant 1 à 6 atomes de carbone, phényle, benzyle ou acétyle,

$R^3$ est l'hydrogène, un radical alkyle inférieur ayant 1 à 6 atomes de carbone ou un radical phényle, et

$R^4$ est l'hydrogène, un radical alkyle inférieur, alkoxy inférieur ayant chacun 1 à 6 atomes de carbone, le fluor, le chlore, le brome, un radical trifluorométhoxy, phényle, phénoxy, benzyle, phénéthyle, benzyloxy, acétyle ou un groupe de formule

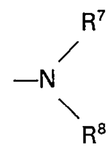

dans laquelle

$R^7$ et $R^8$ sont identiques ou différents et représentent l'hydrogène, un groupe alkyle inférieur ayant 1 à 6 atomes de carbone, phényle, benzyle ou acétyle.

8. Procédé de préparation de N-sulfonamidoéthylindoles de formule

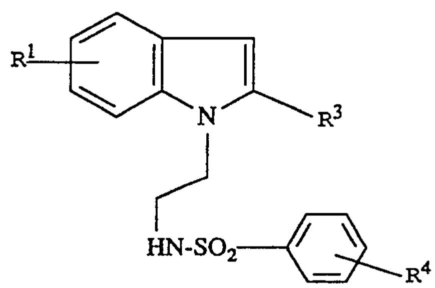

(II)

dans laquelle

$R^1$ représente l'hydrogène, un groupe alkyle inférieur, alkoxy inférieur ayant chacun 1 à 6 atomes de carbone, le fluor, le chlore, le brome, l'iode, un groupe trifluorométhyle, trifluorométhoxy, phényle, phénoxy, benzyle, phénéthyle, phénoxy, benzyloxy, acétyle ou un groupe de formule

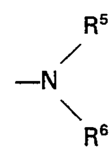

dans laquelle

$R^5$ et $R^6$ sont identiques ou différents et représentent l'hydrogène, un groupe alkyle inférieur ayant 1 à 6 atomes de carbone, phényle, benzyle ou acétyle,

$R^3$ est l'hydrogène, un radical alkyle inférieur ayant 1 à 6 atomes de carbone ou un radical phényle, et

$R^4$ est l'hydrogène, un radical alkyle inférieur, alkoxy inférieur ayant chacun 1 à 6 atomes de carbone, le fluor, le chlore, le brome, un radical trifluorométhoxy, phényle, phénoxy, benzyle, phénéthyle, benzyloxy, acétyle ou un groupe de formule

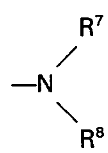

dans laquelle

$R^7$ et $R^8$ sont identiques ou différents et représentent l'hydrogène, un groupe alkyle inférieur ayant 1 à 6 atomes de carbone, phényle, benzyle ou acétyle, caractérisé en ce qu'on fait réagir des indoles de formule générale (IV)

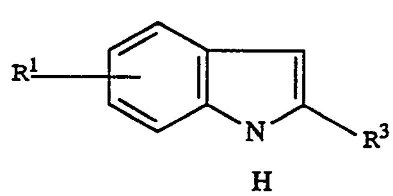

(IV)

dans laquelle

$R^1$ et $R^3$ ont la définition indiquée ci-dessus, avec des aziridines de formule générale (V)

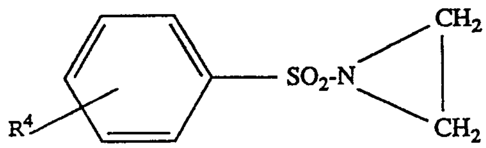

(V)

dans laquelle

$R^4$ a la définition indiquée ci-dessus, en présence de bases dans des solvants inertes.

## Claims

1. Indolylpropionic acids of the formula

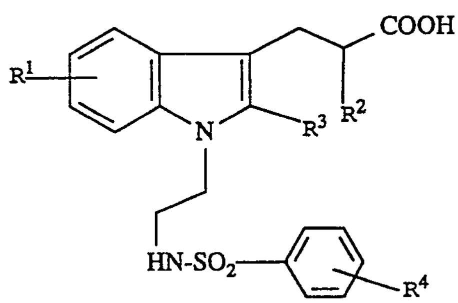

(I)

in which

$R^1$ represents hydrogen, lower alkyl, lower alkoxy with in each case 1 to 6 C atoms, fluorine, chlorine, bromine, iodine, trifluoromethyl, trifluoromethoxy, phenyl, phenoxy, benzyl, phenethyl, benzyloxy, acetyl or a group of the formula

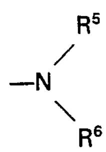

in which

$R^5$ and $R^6$ are identical or different and denote hydrogen, lower alkyl with 1 to 6 C atoms, phenyl, benzyl or acetyl,

$R^2$ represents hydrogen or lower alkyl with 1 to 6 C atoms,

$R^3$ represents hydrogen, lower alkyl with 1 to 6 C atoms or phenyl, and

$R^4$ represents hydrogen, lower alkyl, lower alkoxy with in each case 1 to 6 C atoms, fluorine, chlorine, bromine, trifluoromethoxy, phenyl, phenoxy, benzyl, phenethyl, benzyloxy, acetyl or a group of the formula

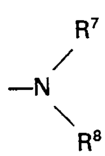

in which

$R^7$ and $R^8$ are identical or different and denote hydrogen, lower alkyl with 1 to 6 C atoms, phenyl, benzyl or acetyl, and the salts thereof.

2. Indolylpropionic acids according to Claim 1, in which

$R^1$ represents hydrogen, $C_1$—$C_4$-alkyl, $C_1$—$C_4$-alkoxy, fluorine, chlorine, bromine, trifluoromethyl, $C_1$—$C_4$-alkylamino, or di-$C_1$—$C_4$-alkylamino,

$R^2$ represents hydrogen or $C_1$—$C_4$-alkyl,

$R^3$ represents hydrogen, $C_1$—$C_4$-alkyl or phenyl, and

$R^4$ represents hydrogen, $C_1$—$C_4$-alkyl, $C_1$—$C_4$-alkoxy, fluorine, chlorine, bromine, trifluoromethyl, $C_1$—$C_4$-alkylamino, or di-$C_1$—$C_4$-alkylamino, and the salts thereof.

3. Indolylpropionic acids of the formula I according to Claim 1 for use in the therapeutic treatment of diseases.

4. Process for the preparation of indolylpropionic acids of the general formula (I)

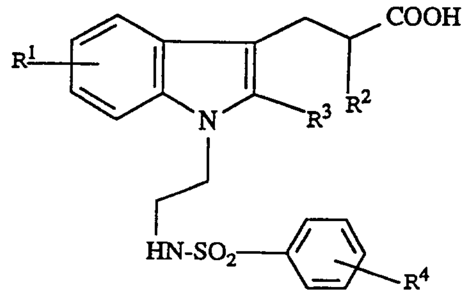

(I)

in which

$R^1$ represents hydrogen, lower alkyl, lower alkoxy with in each case 1 to 6 C atoms, fluorine, chlorine, bromine, iodine, trifluoromethyl, trifluoromethoxy, phenyl, phenoxy, benzyl, phenethyl, benzyloxy, acetyl or a group of the formula

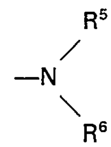

in which

$R^5$ and $R^6$ are identical or different and denote hydrogen, lower alkyl with 1 to 6 C atoms, phenyl, benzyl or acetyl,

$R^2$ represents hydrogen or lower alkyl with 1 to 6 C atoms,

$R^3$ represents hydrogen or lower alkyl with 1 to 6 C atoms or phenyl, and

$R^4$ represents hydrogen, lower alkyl, lower alkoxy with in each case 1 to 6 C atoms, fluorine, chlorine, bromine, trifluoromethoxy, phenyl, phenoxy, benzyl, phenethyl, benzyloxy, acetyl or a group of the formula

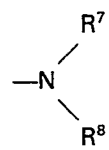

in which

$R^7$ and $R^8$ are identical or different and denote hydrogen, lower alkyl with 1 to 6 C atoms, phenyl, benzyl or acetyl, and the salts thereof, which is characterized in that N-sulphonamidoethyl-indoles of the general formula (II)

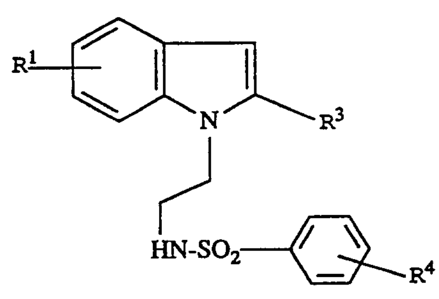

(II)

in which

$R^1$, $R^3$ and $R^4$ have the meaning specified, are reacted with acrylic acids of the general formula (III)

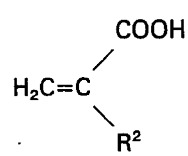

(III)

in which

$R^2$ has the abovementioned meaning, in inert solvents, and, in the case of the preparation of the salts, are reacted with an appropriate base.

5. Medicaments containing indolylpropionic acids of the formula I according to Claim 1.

6. Use of indolylpropionic acids of the formula I according to Claim 1 for the preparation of medicaments having a thrombocyte aggregation-inhibiting and/or thromboxane $A_2$ antagonistic action.

7. N-sulphonamidoethyl-indoles of the formula

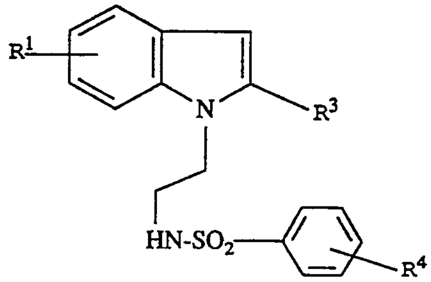

(II)

in which

$R^1$ represents hydrogen, lower alkyl, lower alkoxy with in each case 1 to 6 C atoms, fluorine, chlorine, bromine, iodine, trifluoromethyl, trifluoromethoxy, phenyl, phenoxy, benzyl, phenethyl, benzyloxy, acetyl or a group of the formula

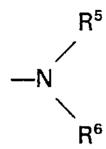

in which

$R^5$ and $R^6$ are identical or different and denote hydrogen, lower alkyl with 1 to 6 C atoms, phenyl, benzyl or acetyl,

$R^3$ represents hydrogen, lower alkyl with 1 to 6 C atoms or phenyl, and

$R^4$ represents hydrogen, lower alkyl, lower alkoxy with in each case 1 to 6 C atoms, fluorine, chlorine, bromine, trifluoromethoxy, phenyl, phenoxy, benzyl, phenethyl, benzyloxy, acetyl or a group of the formula

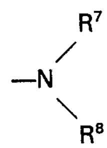

in which

$R^7$ and $R^8$ are identical or different and denote hydrogen, lower alkyl with 1 to 6 C atoms, phenyl, benzyl or acetyl.

8. Process for the preparation of N-sulphonamidoethyl-indoles of the formula

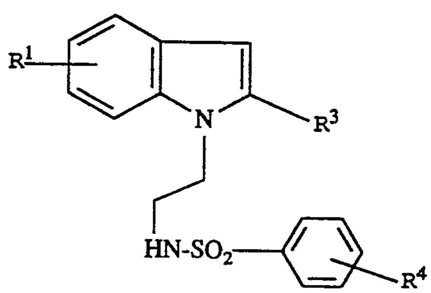

(II)

in which

$R^1$ represents hydrogen, lower alkyl, lower alkoxy with in each case 1 to 6 C atoms, fluorine, chlorine, bromine, iodine, trifluoromethyl, trifluoromethoxy, phenyl, phenoxy, benzyl, phenethyl, benzyloxy, acetyl or a group of the formula

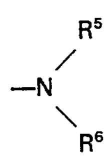

in which

$R^5$ and $R^6$ are identical or different and denote hydrogen, lower alkyl with 1 to 6 C atoms, phenyl, benzyl or acetyl,

$R^3$ represents hydrogen, lower alkyl with 1 to 6 C atoms or phenyl, and

$R^4$ represents hydrogen, lower alkyl, lower alkoxy with in each case 1 to 6 C atoms, fluorine, chlorine, bromine, trifluoromethoxy, phenyl, phenoxy, benzyl, phenethyl, benzyloxy, acetyl or a group of the formula

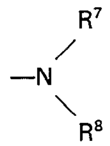

in which

$R^7$ and $R^8$ are identical or different and denote hydrogen, lower alkyl with 1 to 6 C atoms, phenyl, benzyl or acetyl, characterized in that indoles of the general formula (IV)

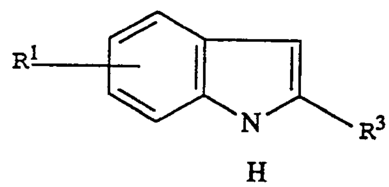

(IV)

in which

$R^1$ and $R^3$ have the abovementioned meaning, are reacted with aziridines of the general formula (V)

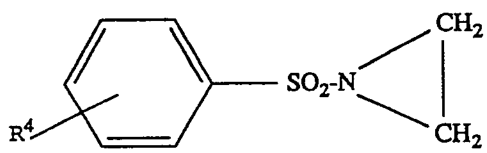

(V)

in which

$R^4$ has the abovementioned meaning, in the presence of bases in inert solvents.